Europäisches Patentamt

European Patent Office

Office européen des brevets

(19)

(11) Veröffentlichungsnummer: **0 300 316 B1**

(12) **EUROPÄISCHE PATENTSCHRIFT**

(45) Veröffentlichungstag der Patentschrift: **30.09.92**

(21) Anmeldenummer: **88111063.9**

(22) Anmeldetag: **11.07.88**

(51) Int. Cl.⁵: **A61B 6/00**, F16M 11/26

(54) **Stativ für ein Röntgenuntersuchungsgerät mit einer Teleskopsäule.**

(30) Priorität: **23.07.87 DE 8710117 U**

(43) Veröffentlichungstag der Anmeldung:
**25.01.89 Patentblatt 89/04**

(45) Bekanntmachung des Hinweises auf die
Patenterteilung:
**30.09.92 Patentblatt 92/40**

(84) Benannte Vertragsstaaten:
**BE DE FR GB**

(56) Entgegenhaltungen:
**BE-A- 531 154**
**BE-A- 899 228**
**DE-C- 831 773**
**US-A- 3 902 070**

(73) Patentinhaber: **SIEMENS AKTIENGESELL-
SCHAFT**
**Wittelsbacherplatz 2**
**W-8000 München 2(DE)**

(72) Erfinder: **Heinz, Lothar, Dipl.-Ing. (FH)**
**Weingasse 63**
**W-8524 Neunkirchen(DE)**
Erfinder: **Schmitt, Thomas, Dipl.-Ing. (FH)**
**Hans-Sebald-Strasse 2**
**W-8550 Forchheim(DE)**

## Beschreibung

Die Erfindung betrifft ein Stativ für ein Röntgenuntersuchungsgerät mit einer Teleskopsäule, die ein Teil des Röntgenuntersuchungsgerätes, z.B. einen Röntgenstrahler, in Richtung ihrer Längsachse verstellbar führt und wenigstens drei ineinandergefügte rohrförmige Teleskopteile aufweist, von denen eines relativ zu den anderen ortsfest ist, eines das Teil des Röntgenuntersuchungsgerätes trägt und eines mit Mitteln zum Verstellen des Teiles verbunden ist, wobei die Teleskopteile derart miteinander gekoppelt sind, daß beim Verstellen des Teiles zwischen jeweils zwei einander benachbarten Teleskopteilen die gleiche Relativverschiebung auftritt, und zwischen einander benachbarten Teleskopteilen jeweils Anschlagmittel zur Begrenzung der Relativverschiebung vorgesehen sind.

Ein solches Stativ, das als Deckenstativ mit vertikal gerichteter Teleskopsäule ausgeführt ist, an der ein Röntgenstrahler höhenverstellbar angebracht ist, ist aus der US-PS 3 902 070 bekannt. Als Mittel zum Verstellen des Röntgenstrahlers sowie zur Koppelung der Teleskopteile miteinander sind bei dem bekannten Stativ Seile vorgesehen. Dabei müssen diejenigen Seile, die zur Koppelung der Teleskopteile dienen, eine solche Länge aufweisen, daß beim vollständigen Ausziehen oder Zusammenschieben der Teleskopsäule alle zur Begrenzung der Relativverschiebung zwischen einander benachbarten Teleskopteilen vorgesehenen Anschlagmittel im wesentlichen zur gleichen Zeit wirksam werden, da andernfalls beim ruckartigen vollständigen Ausziehen oder Zusammenschieben der Teleskopsäule die die Teleskopteile koppelnden Seile reißen könnten. Im Zuge der Montage des bekannten Stativs muß daher die Teleskopsäule in der Regel mehrfach zur Korrektur der Längen der die Teleskopteile koppelnden Seile demontiert werden, bis diese schließlich so abgestimmt sind, daß alle Anschlagmittel gleichzeitig wirksam werden. Die gleiche Prozedur, die umständlich, zeitraubend und kostenintensiv ist, ist erforderlich, wenn im Wartungs- oder Reparaturfalle einzelne Seile ausgetauscht werden müssen.

Der Erfindung liegt die Aufgabe zugrunde, ein Stativ der eingangs genannten Art so auszubilden, daß Zusammenbau, Wartung und Reparatur der Teleskopsäule auf einfache Weise möglich sind.

Nach der Erfindung wird diese Aufgabe dadurch gelöst, daß Einstellmittel vorgesehen sind, mittels derer die Lage der Teleskopteile in Richtung der Längsachse der Teleskopsäule relativ zueinander derart einstellbar ist, daß alle Anschlagmittel im wesentlichen gleichzeitig wirksam werden, und daß die Einstellmittel derart ausgebildet und angeordnet sind, daß sie ohne Demontage der Teleskopsäule betätigbar sind. Infolge der Erfindung kann somit die Teleskopsäule bei der Montage des Stativs ohne besondere Maßnahmen im Hinblick auf die Koppelung der Teleskopteile zusammengebaut werden, da es mittels der Einstellmittel, die ohne Demontage der Teleskopsäule nach erfolgtem Zusammenbau betätigbar sind, möglich ist, die Lage der Teleskopteile in Richtung der Längsachse der Teleskopsäule relativ zueinander so einzustellen, daß alle Anschlagmittel beim vollständigen Ausziehen und/oder Zusammenschieben der Teleskopsäule gleichzeitig wirksam werden.

Im Falle eines Stativs, bei dem von jeweils drei einander benachbarten Teleskopteilen der Teleskopsäule das innere mit dem äußeren durch ein Zugorgan, z.B. ein Seil, gekoppelt ist, das über eine an dem mittleren Teleskopteil angebrachte Umlenkrolle geführt ist, derart, daß die von dem inneren und dem äußeren Teleskopteil zu der Umlenkrolle führenden Abschnitte des Zugorgans parallel zueinander verlaufen, ist nach einer Variante der Erfindung vorgesehen, daß Einstellmittel jeweils drei einander benachbarten Teleskopteilen zugeordnet sind. Dabei können die Einstellmittel durch Mittel zum Verändern der Länge des Zugorgans oder durch Mittel zum Verschieben eines der Befestigungspunkte des Zugorgans an dem äußeren oder inneren Teleskopteil in Richtung der Längsachse der Teleskopsäule gebildet sein. Nach einer bevorzugten Ausführungsform der Erfindung ist jedoch vorgesehen, daß die Einstellmittel jeweils durch Mittel zum Verschieben der Umlenkrolle in Richtung der Längsachse der Teleskopsäule gebildet sind. Dies ist deshalb von Vorteil, weil ohnehin konstruktive Maßnahmen zur Befestigung der Umlenkrolle getroffen werden müssen und die Einstellmittel so auf einfache Weise zu verwirklichen sind. So ist nach einer Variante der Erfindung vorgesehen, daß die Umlenkrolle jeweils an einem Tragteil angebracht ist, das mittels einer Stellschraube verstellbar an dem mittleren dreier benachbarter Teleskopteile gehalten ist, wobei zwischen dem Tragteil und dem mittleren Teleskopteil zur Ausschaltung von Spiel eine Druckfeder angeordnet sein kann.

Eine Ausführungsform der Erfindung sieht vor, daß die Anschlagmittel an den Teleskopteilen derart angebracht sind, daß bei vollständig zusammengeschobener Teleskopsäule die dem Teil des Röntgenuntersuchungsgerätes zugewandten Enden aller Teleskopteile in einer Ebene liegen. Infolge dieser Maßnahme gestaltet sich der Zusammenbau der Teleskopsäule besonders einfach, da zur korrekten Einstellung der Teleskopsäule die Einstellmittel lediglich derart betätigt werden müssen, daß bei vollständig zusammengeschobener Teleskopsäule die erwähnten Enden der Teleskopteile in einer gemeinsamen Ebene liegen.

In den beigefügten Zeichnungen ist ein Ausführungsbeispiel der Erfindung dargestellt. Es zeigen:

Fig. 1 eine perspektivische Ansicht eines erfindungsgemäßen Stativs,

Fig. 2 in schematischer Darstellung einen Längsschnitt durch die Teleskopsäule des erfindungsgemäßen Stativs,

Fig. 3 die Einzelheit A gemäß Fig. 2 in gegenüber Fig. 2 vergrößerter Darstellung, und

Fig. 4 einen teilweisen Längsschnitt durch die Teleskopsäule des erfindungsgemäßen Stativs in vollständig zusammengeschobenem Zustand.

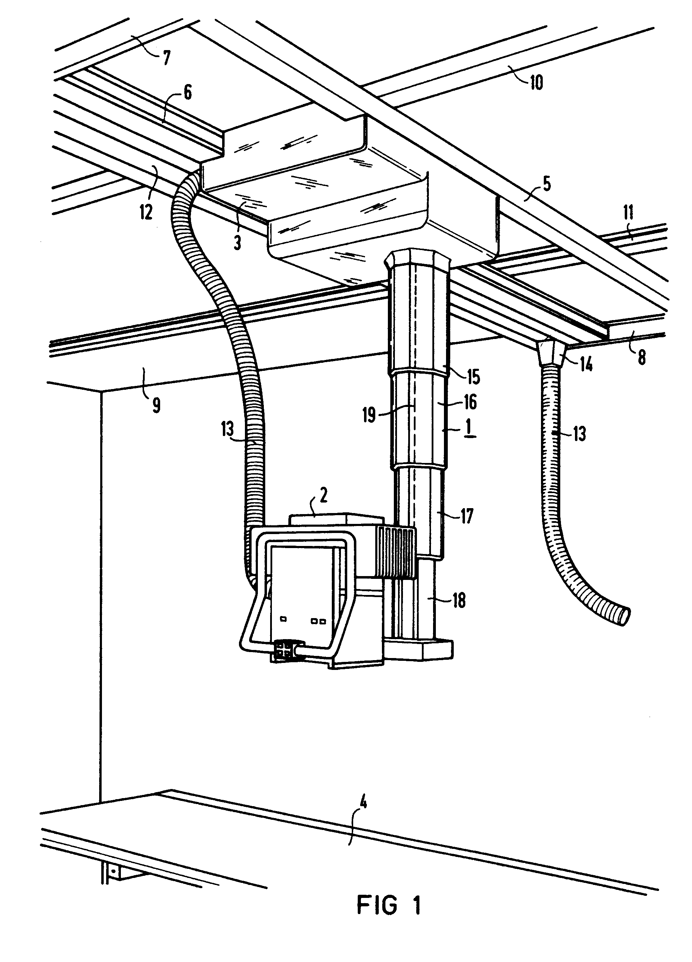

Die Fig. 1 zeigt ein Stativ für ein Röntgenuntersuchungsgerät, das als Deckenstativ mit einer vertikalen Teleskopsäule 1 ausgeführt ist, die ein Teil des Röntgenuntersuchungsgerätes, nämlich einen Röntgenstrahler 2 höhenverstellbar führt. Die Teleskopsäule 2 ist an einem Wagen 3 oberhalb eines Untersuchungstisches 4 angebracht. Der Wagen 3 ist in parallelen Schienen 5, 6 mittels nicht sichtbarer Rollen quer zu dem Untersuchungstisch 4 verschiebbar. Die mittels der Endstücke 7 und 8 miteinander verbundenen Schienen 5 und 6 sind ihrerseits mittels ebenfalls nicht sichtbarer Rollen in an der Decke 9 des Untersuchungsraumes angebrachten, im rechten Winkel zu den Schienen 5 und 6 verlaufenden parallelen Deckenschieben 10 und 11 verfahrbar, so daß der Röntgenstrahler 2 auch längs des Untersuchungstisches 4 verschiebbar ist. Parallel zu der Schiene 6 erstreckt sich ein Kanal 12, in dem ein flexibles Versorgungskabel 13 verläuft, das durch eine in bezug auf den Wagen 3 stationäre, im Bereich des Endstückes 8 vorgesehene Kabeldurchführung 14 in den Kanal 12 eintritt, im Bereich des Wagens 3 aus diesem austritt und zu dem Röntgenstrahler 2 geführt ist. Die Teleskopsäule 1 besteht aus in herkömmlicher Weise ineinandergefügten rohrförmigen Teleskopteilen 15, 16, 17 und 18, wobei das Teleskopteil 15 fest an dem Wagen 3 angebracht und somit in Richtung der Längsachse der Teleskopsäule 2 relativ zu den anderen Teleskopteilen 16, 17 und 18 ortsfest ist, während das Teleskopteil 18 den Röntgenstrahler 2 trägt und außerdem mit einem in Fig. 1 strichliert eingetragenen Seil 19 verbunden ist, das zur Höhenverstellung des Röntgenstrahlers 2 auf einer in den Fig. nicht dargestellte Seiltrommel auf- und abrollbar ist.

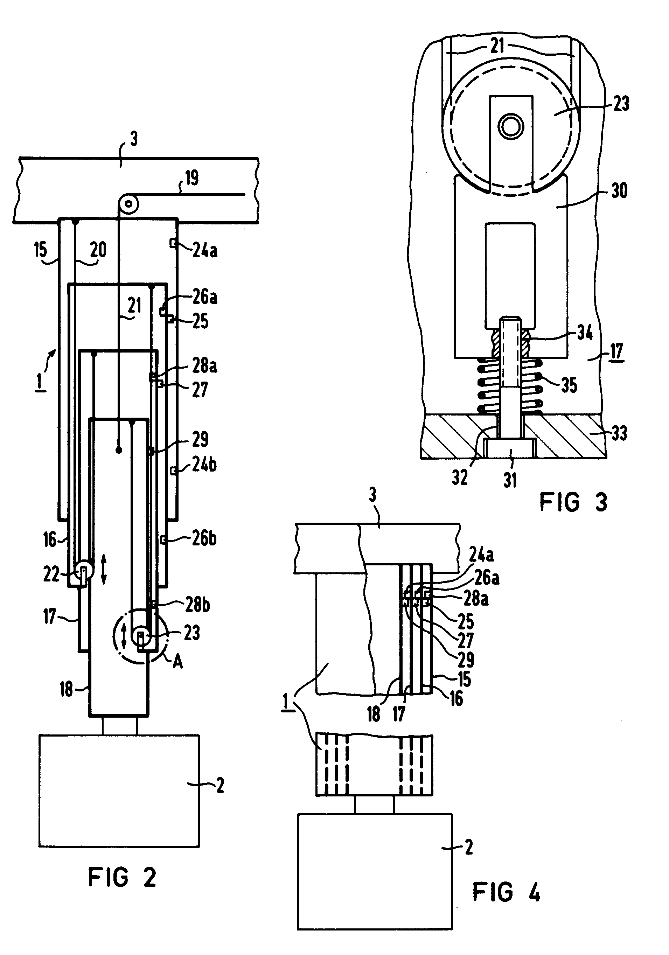

Wie aus Fig. 2 ersichtlich ist, sind die Teleskopteile 15, 16, 17 und 18 derart miteinander gekoppelt, daß beim Verstellen des Röntgenstrahlers 2 mittels des Seiles 19 zwischen jeweils zwei einander benachbarten Teleskopteilen 15 und 16, 16 und 17, 17 und 18 jeweils die gleiche Relativverschiebung auftritt. Im einzelnen wird dies durch Zugorgane, nämlich die Seile 20 und 21 bewirkt, die jeweils drei einander benachbarte Teleskopteile, nämlich 15, 16 und 17 sowie 16, 17 und 18 miteinander koppeln. Dabei sind die Seile 20 bzw. 21 mit ihren Enden jeweils an den inneren und äußeren Teleskopteilen 17 und 15 bzw. 18 und 16 jeweils dreier einander benachbarter Teleskopteile 15, 16 und 17 bzw. 16, 17 und 18 im Bereich von deren in Fig. 2 oberen Enden befestigt. An dem jeweils mittleren Teleskopteil 16 bzw. 17 ist eine Umlenkrolle 22 bzw. 23 befestigt, über die das Seil 20 bzw. 21 derart geführt ist, daß die von dem jeweils inneren Teleskopteil 17 bzw. 18 zu dem äußeren Teleskopteil 15 bzw. 16 führenden Abschnitte der Seile 20 bzw. 21 parallel zueinander verlaufen.

Zwischen einander benachbarten Teleskopteilen 15 und 16, 16 und 17, 17 und 18 sind zur Begrenzung der zwischen diesen beim vollständigen Ausziehen bzw. Zusammenschieben der Teleskopsäule 1 auftretenden Relativverschiebungen Anschläge 24a, 24b und 25, 26a, 26b und 27, 28a, 28b und 29 vorgesehen. Dabei sind die Anschläge 24a, 26a und 28a jeweils an den oberen Enden und die Anschläge 24b, 26b und 28b jeweils an den unteren Enden der inneren Wandungen der Teleskopteile 15, 16 und 17 im gleichen Abstand voneinander angebracht, während die Anschläge 25, 27 und 29 jeweils an den äußeren Wandungen der Teleskopteile 16, 17 und 18 im Bereich von deren oberen Enden vorgesehen sind.

Um sicherstellen zu können, daß die Anschläge 25, 27 und 29 beim vollständigen Ausziehen der Teleskopsäule 1 mit den Anschlägen 24b, 26b und 28b und beim vollständigen Zusammenschieben der Teleskopsäule 1 mit den Anschlägen 24a, 26a und 26b im wesentlichen gleichzeitig in Eingriff kommen, was erforderlich ist, um unzulässige Belastungen von den Seilen 20 und 21 fernzuhalten, sind jeweils drei einander benachbarten Teleskopteilen 15, 16 und 17 bzw. 16, 17 und 18 Einstellmittel zugeordnet, mittels derer diese in ihrer Lage relativ zueinander in der für das gleichzeitige Wirksamwerden der Anschläge erforderlichen Weise verstellbar sind. Zu diesem Zweck sind die Umlenkrollen 22 und 23 in Richtung der Längsachse der Teleskopsäule 1 verschiebbar an den Teleskopteilen 16 und 17 angebracht, wie dies in Fig. 2 durch den Umlenkrollen 22 und 23 zugeordnete Doppelpfeile symbolisiert ist. Im einzelnen sind die Umlenkrollen 22 und 23, wie dies in Fig. 3 am Beispiel der an dem Teleskopteil 17 angebrachten Umlenkrolle 23 dargestellt ist, an einem Tragteil 30 angebracht, das mittels einer Stellschraube 31 verstellbar an dem Teleskopteil 17 gehalten ist. Dabei erstreckt sich die Stellschraube 31 durch eine Durchgangsbohrung 32, die in einem an dem dem Röntgenstrahler 2 zugewandten, d.h. dem unteren Ende des Teleskopteiles 17 angebrachten Rand 33 vorgesehen ist, zu dem Tragteil 31, das eine geeignete Gewindebohrung 34 für die Stellschraube 31 aufweist. Da zur Vermeidung von Spiel zwischen dem Tragteil 30 und dem Teleskopteil 17 eine Druckfeder 35 vorgesehen ist, kann das Tragteil 30 durch Verdrehen der Stellschraube 31 in Richtung der Längsachse der Teleskopsäule 1 verstellt werden. Da die zu der Umlenkrolle 23 gehörige Stellschraube 31 und die entsprechende Stellschraube der Umlenkrolle 22 somit auch bei vollständig zusammengebauter Teleskopsäule ohne weiteres zugänglich ist, können die Einstellarbeiten an den Umlenkrollen 22 und 23, die erforderlich sind, um zu gewährleisten, daß die Anschläge in der oben beschriebenen Weise gleichzeitig wirksam werden, nach erfolgtem Zusammenbau der Teleskopsäule 1 bzw. des gesamten Statives erfolgen.

Die erforderliche Einstellung der Umlenkrollen 22 und 23 gestaltet sich dann besonders einfach, wenn die Anschläge 24a, 26a, 28a und 25, 26, 27 derart an den Teleskopteilen 15 bis 18 angebracht sind, daß beim Wirksamwerden dieser Anschläge, also bei vollständig zusammengeschobener Teleskopsäule 1 die dem Röntgenstrahler 2 zugewandten Enden aller Teleskopteile 15 bis 18 in einer gemeinsamen Ebene liegen, wie dies in Fig. 4 angedeutet ist, da dann die Stellschrauben lediglich so betätigt werden müssen, daß sich die Enden der Teleskopteile 15 bis 18 wie erwähnt in einer gemeinsamen Ebene befinden. Wenn an den Teleskopteilen 15 bis 17 die Anschläge 24a und 24b, 26a und 26b, 28a und 28b wie oben erwähnt jeweils den gleichen Abstand voneinander aufweisen, ist zugleich sichergestellt, daß auch bei dem vollständigen Ausziehen der Teleskopsäule 1 die Anschläge 24b und 25, 26b und 27, 28b und 29 gleichzeitig wirksam werden.

**Patentansprüche**

1. Stativ für ein Röntgenuntersuchungsgerät mit einer Teleskopsäule (1), die ein Teil des Röntgenuntersuchungsgerätes, z.B. einen Röntgenstrahler (2), in Richtung ihrer Längsachse verstellbar führt und wenigstens drei ineinandergefügte rohrförmige Teleskopteile (15, 16, 17, 18) aufweist, von denen eines (15) relativ zu den anderen ortsfest ist, eines (18) das Teil (2) des Röntgenuntersuchungsgerätes trägt und eines (18) mit Mitteln (19) zum Verstellen des Teiles (2) verbunden ist, wobei die Teleskopteile (15, 16, 17, 18) derart miteinander gekoppelt sind, daß beim Verstellen des Teiles (2) zwischen jeweils zwei einander benachbarten Teleskopteilen (15 und 16, 16 und 17, 17 und 18) jeweils die gleiche Relativverschiebung auftritt, und zwischen einander benachbarten Teleskopteilen (15 und 16, 16 und 17, 17 und 18) jeweils Anschlagmittel (24a, 24b und 25, 26a, 26b und 27, 28a, 28b und 29) zur Begrenzung der Relativverschiebung vorgesehen sind, **dadurch gekennzeichnet,** daß Einstellmittel (30, 31) vorgesehen sind, mittels derer die Lage der Teleskopteile (15, 16, 17, 18) in Richtung der Längsachse der Teleskopsäule (1) relativ zueinander derart einstellbar ist, daß alle Anschlagmittel (24a, 25, 26a, 27, 28a, 29 bzw. 24b, 25, 26b, 27, 28b, 29) im wesentlichen gleichzeitig wirksam werden, und daß die Einstellmittel (30, 31) derart ausgebildet und angeordnet sind, daß sie ohne Demontage der Teleskopsäule (1) betätigbar sind.

2. Stativ nach Anspruch 1, bei dem von jeweils drei einander benachbarten Teleskopteilen (15, 16, 17; 16, 17, 18) der Teleskopsäule (1) das innere (17 bzw. 18) mit dem äußeren (15 bzw. 16) durch ein Zugorgan (20 bzw. 21) gekoppelt ist, das über eine an dem mittleren Teleskopteil (16 bzw. 17) angebrachte Umlenkrolle (22 bzw. 23) geführt ist, derart, daß die von dem inneren (17 bzw. 18) und dem äußeren (15 bzw. 16) Teleskopteil zu der Umlenkrolle (22 bzw. 23) führenden Abschnitte des Zugorgans (20 bzw. 21) parallel zueinander verlaufen, **dadurch gekennzeichnet,** daß Einstellmittel (30, 31) jeweils drei einander benachbarten Teleskopteilen (15, 16, 17 bzw. 16, 17, 18) zugeordnet sind.

3. Stativ nach Anspruch 2, **dadurch gekennzeichnet,** daß die Einstellmittel jeweils durch Mittel (30, 31) zum Verschieben der Umlenkrolle (22 bzw. 23) in Richtung der Längsachse der Teleskopsäule (1) gebildet sind.

4. Stativ nach Anspruch 3, **dadurch gekennzeichnet,** daß die Umlenkrolle (22 bzw. 23) jeweils an dem dem Teil (2) des Röntgenuntersuchungsgerätes zugewandten Ende des mittleren (16 bzw. 17) dreier benachbarter Teleskopteile (15, 16, 17 bzw. 16, 17, 18) angeordnet ist.

5. Stativ nach Anspruch 3 oder 4, **dadurch gekennzeichnet,** daß die Umlenkrolle (22 bzw. 23) jeweils an einem Tragteil (30) angebracht ist, das mittels einer Stellschraube (31) verstellbar an dem mittleren (16 bzw. 17) dreier benachbarter Teleskopteile (15, 16, 17 bzw. 16, 17, 18) gehalten ist.

6. Stativ nach Anspruch 5, **dadurch gekennzeichnet,** daß zwischen dem Tragteil (30) und dem mittleren Teleskopteil (16, 17) zur Ausschaltung von Spiel eine Druckfeder (35) angeordnet ist.

**7.** Stativ nach einem der Ansprüche 1 bis 6, **dadurch gekennzeichnet,** daß die Anschlagmittel (24a, 25, 26a, 27, 28a, 29) an den Teleskopteilen (15, 16, 17, 18) derart angeordnet sind, daß bei vollständig zusammengeschobener Teleskopsäule (1) die dem Teil (2) des Röntgenuntersuchungsgerätes zugewandten Enden aller Teleskopteile (15, 16, 17, 18) in einer gemeinsamen Ebene liegen, wenn alle Anschläge (24a, 25, 26a, 27, 28a, 29) wirksam sind.

**Claims**

**1.** Support for an X-ray examination device with a telescopic column (1), which guides a part of the X-ray examination device, e.g. an X-radiator (2), adjustably in the direction of its longitudinal axis and has at least three tubular telescope parts (15, 16, 17, 18) inserted one within the other, one (15) of which is stationary relative to the others, one (18) carries the part (2) of the X-ray examination device, and one (18) is connected to means (19) for adjusting the part (2), the telescope parts (15, 16, 17, 18) being coupled to one another in such a way that when the part (2) is adjusted the same relative displacement respectively occurs between each two telescope parts (15 and 16, 16 and 17, 17 and 18) adjacent to one another, and stop means (24a, 24b and 25, 26a, 26b and 27, 28a, 28b and 29) for limiting the relative displacement being respectively provided between telescope parts (15 and 16, 16 and 17, 17 and 18) adjacent to one another, characterised in that adjustment means (30, 31) are provided, by means of which the position of the telescope parts (15, 16, 17, 18) can be adjusted relative to one another in the direction of the longitudinal axis of the telescopic column (1) in such a way that all the stop means (24a, 25, 26a, 27, 28a, 29 and 24b, 25, 26b, 27, 28b, 29) take effect at substantially the same time, and in that the adjustment means (30, 31) are constructed and arranged so as to be actuatable without dismantling the telescopic column (1).

**2.** A support according to claim 1, wherein of three respectively adjacent telescope parts (15, 16, 17; 16, 17, 18) of the telescopic column (1) the inner one (17 or 18) is coupled to the outer one (15 or 16) by means of a traction element (20 or 21) which is guided over a deflection roller (22 or 23) attached to the middle telescope part (16 or 17) such that the sections of the traction element (20 or 21) leading from the inner (17 or 18) and the outer (15 or 16) telescope part to the deflection roller (22 or 23) extend parallel to each other, characterised in that adjustment means (30, 31) are respectively provided for three telescope parts (15, 16, 17 or 16, 17, 18) adjacent to one another.

**3.** A support according to claim 2, characterised in that the adjustment means are respectively formed by means (30, 31) for displacing the deflection roller (22 or 23) in the direction of the longitudinal axis of the telescopic column (1).

**4.** A support according to claim 3, characterised in that the deflection roller (22 or 23) is arranged respectively at the end of the middle (16 or 17) of three adjacent telescope parts (15, 16, 17 or 16, 17, 18) facing the part (2) of the X-ray examination device.

**5.** A support according to claim 3 or 4, characterised in that the deflection roller (22 or 23) is respectively attached to a carrying part (30) that is held adjustably, by means of an adjusting screw (31), at the middle (16 or 17) of three adjacent telescope parts (15, 16, 17 or 16, 17, 18).

**6.** A support according to claim 5, characterised in that a compression spring (35) is arranged between the carrying part (30) and the middle telescope part (16, 17) in order to suppress play.

**7.** A support according to one of claims 1 to 6, characterised in that the stop means (24a, 25, 26a, 27, 28a, 29) are arranged at the telescope parts (15, 16, 17, 18) such that when the telescopic column (1) is completely retracted the ends of all the telescope parts (15, 16, 17, 18) facing the part (2) of the X-ray examination device lie in a common plane when all the stops (24a, 25, 26a, 27, 28a, 29) take effect.

**Revendications**

**1.** Statif pour un appareil de radiodiagnostic comportant une colonne télescopique (1) qui guide, avec possibilité de déplacement dans la direction de son axe longitudinal, une partie de l'appareil de radiodiagnostic, par exemple un émetteur radiologique (2), et possède au moins trois éléments télescopiques tubulaires (15,16,17,18) insérés les uns dans les autres, et dont l'un (15) est fixe par rapport aux autres, dont l'autre (18) porte la partie (2) de l'appareil de diagnostic et dont un autre (18) est raccordé à des moyens (19) pour déplacer la partie (2), les éléments télescopiques (15,16,17,18) étant accouplés entre eux de telle sorte que, lors du déplacement de l'élément (2), le même déplacement relatif apparaît entre deux éléments télescopiques réciproquement voisins (15 et 16, 16 et 17, 17 et 18), et des éléments respectifs de butée (24a, 24b et 15, 26a, 26b et 27, 28a, 28b et 29) servant à limiter le déplacement relatif étant prévus entre des éléments télescopiques réciproquement voisins (15 et 16, 16 et 17, 17 et 13), caractérisé par le fait qu'il est prévu des moyens de réglage (30, 31) à l'aide desquels la position relative des éléments télescopiques (15,16,17,18) est réglable dans la direction de l'axe longitudinal de la colonne télescopique (1), que tous les éléments de butée (24a,25,26a,27,28a,29 ou 24b,25,26b,27,28b,29) deviennent actifs sensiblement simultanément et que les moyens de réglage (30,31) sont agencés et disposés de telle sorte qu'ils peuvent être actionnés sans démontage de la colonne télescopique (1).

2. Statif suivant la revendication 1, dans lequel parmi respectivement trois éléments télescopiques réciproquement voisins (15,16,17; 16,17,18) de la colonne télescopique (1), l'élément intérieur (17 ou 18) est couplé à un élément extérieur (15 ou 16) par un organe de traction (20 ou 21), qui est guidé par l'intermédiaire d'un galet de renvoi (22 ou 23) monté sur l'élément télescopique médian (16 ou 17) de telle sorte que les sections de l'organe de traction (20 ou 21), qui s'étendent de l'élément télescopique intérieur (17 ou 18) et de l'élément télescopique extérieur (15 ou 16) jusqu'au galet de renvoi (22 ou 23), sont parallèles entre elles, caractérisé par le fait que des moyens de réglage (30, 31) sont associés respectivement à trois éléments télescopiques réciproquement voisins (15,16,17 ou 16,17,18).

3. Statif suivant la revendication 2, caractérisé par le fait que les moyens de réglage sont formés respectivement par des moyens (30,31) servant à déplacer les galets de renvoi (22 ou 23) dans la direction de l'axe longitudinal de la colonne télescopique (1).

4. Statif suivant la revendication 3, caractérisé par le fait que le galet de renvoi (22 ou 23) est monté sur l'extrémité, tournée vers la partie (2) de l'appareil de radiodiagnostic, de l'élément médian (16 ou 17) qui fait partie de trois éléments télescopiques voisins (15,16,17 ou 16,17,18).

5. Statif suivant la revendication 3 ou 4, caractérisé par le fait que le galet de renvoi (22 ou 23) est monté sur une partie de support (30), qui est maintenue, en étant réglable au moyen d'une vis de réglage (31), sur l'élément médian (16 ou 17) faisant partie de trois éléments télescopiques voisins (15,16,17 ou 16,17,18).

6. Statif suivant la revendication 5, caractérisé par le fait qu'un ressort de pression (35) est disposé, pour supprimer le jeu, entre l'élément de support (3) et l'élément télescopique médian (16,17).

7. Statif suivant l'une des revendications 1 à 6, caractérisé par le fait que les moyens de butée (24a,25,26a,27,28a,29) sont disposés sur les éléments télescopiques (15,16,17,18) de telle sorte que, lorsque la colonne télescopique est complètement rétractée, les extrémités, tournées vers la partie (2) de l'appareil de radiodiagnostic, de tous les éléments télescopiques (15,16,17,18) sont situés dans un plan commun, lorsque toutes les butées (24a,25,26A,27,28a,29) sont actives.

FIG 1

EP 0 300 316 B1

FIG 2

FIG 3

FIG 4